# EUROPEAN PATENT SPECIFICATION

(11) **EP 4 290 233 B1**
(45) Date of publication and mention of the grant of the patent: **25.02.2026**
(21) Application number: 23178237.6
(22) Date of filing: 08.06.2023
(51) Int. Cl.: G01N 33/00, G01D 11/24, H05K 9/00

(54) **EMI PROTECTED ENCLOSURE WITH INTEGRAL INDICATOR-LIGHT GUIDES FOR GAS DETECTORS**
EMI-GESCHÜTZTES GEHÄUSE MIT INTEGRIERTEN INDIKATORLICHTLEITERN FÜR GASSENSOREN
ENCEINTE PROTÉGÉE CONTRE LES INTERFÉRENCES ÉLECTROMAGNÉTIQUES AVEC GUIDES DE LUMIÈRE À INDICATEUR INTÉGRÉS POUR DÉTECTEURS DE GAZ

(30) Priority: 08.06.2022 US 202263366023 P
(43) Date of publication of application: 13.12.2023
(73) Proprietor: Carrier Corporation, Palm Beach Gardens, FL 33418 (US)
(72) Inventor: WARNER, Tanner, Minneapolis (US); PENNAZ, Zach, Minneapolis (US)
(74) Representative: Dehns

(56) References cited:
- US-A- 4 814 571
- US-A1- 2003 224 643
- US-A1- 2016 213 033
- US-A1- 2020 095 118

## Description

### BACKGROUND

The invention relates generally to enclosures for gas detectors, more specifically, to electromagnetic interference protected enclosures for gas detectors.

Generally, intrinsically-safe, hazardous location, gas detectors use cemented windows to allow indicator lights to be viewed from outside the device. However, cemented windows may not always be practical.

US2003/0224643 discloses an enclosure for a gas detector, comprising a housing that is configured to house the gas sensor and comprises first threads on an inner wall. A cover is configured to be detachably connected to the housing and comprises second threads on its outer surface.

US2020/0095118 discloses an electromagnetically shielding enclosure for a gas detector.

### BRIEF DESCRIPTION

Viewed from a first aspect, there is provided an enclosure for a gas detector, the enclosure is configured to house a gas sensor. The enclosure comprises a housing configured to house a gas sensor. The housing comprises first threads of an inner wall of the housing. The enclosure further comprises a cover configured to be detachably connected to the housing. The cover comprises: second threads on an outer surface for mating with the first threads; a conductive portion comprising one or more openings and configured for providing a Faraday cage; and a top layer covering the one or more openings and configured for guiding an indicator light from inside the enclosure to outside the enclosure.

The top layer may be overmolded over a portion of the cover.

The top layer may comprise a transparent material.

The top layer may comprise one or more of polymethyl methacrylate (PMMA), polycarbonate, acrylonitrile butadiene styrene (ABS), high density polyethylene (HDPE), nylon, polyetherimide (PEI), polyethylene terephthalate (PET), polypropylene (PP), and/or polystyrene (PS).

Optionally, there is provided a gas detector comprising: a gas sensor and the enclosure described in the first aspect.

The gas sensor may be an intrinsically safe gas sensor.

Viewed from a second aspect, there is provided a method for manufacturing an enclosure for a gas detector comprising providing a gas sensor; providing a housing configured to house the gas sensor, wherein the housing comprises first threads on an inner wall of the housing; and providing a cover configured to be detachably connected to the housing. The cover comprises second threads on an outer surface for mating with the first threads; a conductive portion comprising one or more openings and configured for providing a Faraday cage; and a top layer covering the one or more openings and configured for guiding an indicator light from inside the enclosure to outside the housing.

Various other aspects, features, and advantages of the invention will be apparent through the detailed description of the invention and the drawings attached hereto. It is also to be understood that the following detailed description are examples and not restrictive of the scope of the invention.

### BRIEF DESCRIPTION OF THE DRAWINGS

Certain exemplary embodiments will now be described in greater detail by way of example only and with reference to the accompanying drawings in which:
FIG. 1 shows a cross sectional view of an example of a gas detector.
FIG. 2 shows an example of a gas detector cover.
FIG. 3 shows an example of a gas detector cover with a transparent layer.

### DETAILED DESCRIPTION

In the following description, for the purposes of explanation, numerous specific details are set forth in order to provide a thorough understanding of the embodiments of the invention. It will be appreciated, however, by those having skill in the art that the embodiments of the invention may be practiced without these specific details or with an equivalent arrangement. In other cases, well-known structures and devices are shown in block diagram form in order to avoid unnecessarily obscuring the embodiments of the invention.

The present disclosure describes an electromagnetic interference (EMI) protected enclosure for gas detectors. Generally, intrinsically-safe gas detectors include electrically conductive material, that is typically opaque, for providing a Faraday cage to prevent EMI. The opaque material doesn't, generally, allow light to travel through it. To overcome this issue, current gas detectors include cemented windows to allow the indicator light to be seen from outside the gas detector. However, the cemented windows may be costly and complicated to manufacture. The EMI protected enclosure as described herein may provide a practical lower cost method to protect the gas sensor while allowing the indicator light to be visible from outside the detector. In some embodiments, a transparent (or relatively transparent) material with high internal reflection may be used to guide the light from the sealed interior of the enclosure to the exterior while maintaining a Faraday cage for EMI protection.

The gas detector enclosure includes a cover portion configured to operatively attach to the housing. The cover includes a conductive portion configured to enclose the sensor electronics and provide a Faraday cage. The conductive portion includes one or more openings configured to attenuate electromagnetic signals and allow light to go through. In some embodiments, the conductive portion may be over-molded with a transparent material for protecting the sensor and allowing the status indicator lights to be seen on the detector.

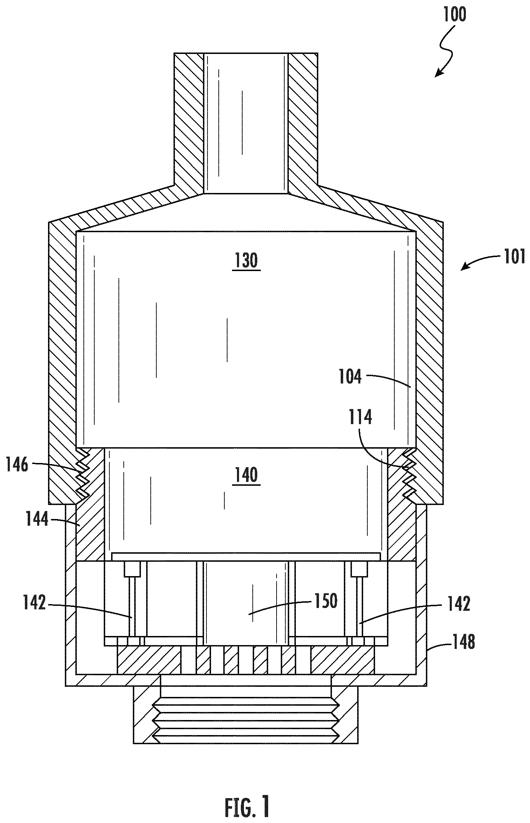

FIG. 1 is a cross sectional view of an example of a gas detector 100, which includes an enclosure 101. The enclosure 101 includes a housing 130 and a cover 140. Enclosure 101 is configured to house one or more components of gas detector 100. A gas sensor 150 is configured to be housed within enclosure 101. For example, gas sensor 150 may be detachably connected to enclosure 101 to facilitate removal of gas sensor 150 (e.g., for maintenance, repair, malfunction, or if gas detector 100 is used to detect a different gas). In some embodiments, gas sensor 150 may be an intrinsically safe (IS) gas sensor having intrinsically safe designed control electronics. In some embodiments, gas sensor 150 may include one or more light indicators 154 configured for communicating a status of the sensor (e.g., operational state, fault, alarm, calibration, etc.)

The cover 140 is configured to be removably connected to housing 130. Cover 140 may house one or more components of gas sensor 150. Sensor 150 is configured to be mounted within enclosure 101 such that it fits between housing 130 and cover 140. The cover 140 includes a highly conductive portion configured to attach to housing 130, and to form an electrical path to create a Faraday cage. The cover 140 includes threads 146 on outer surface 144 for mating with threads 114 on inner wall 104 of enclosure 101.

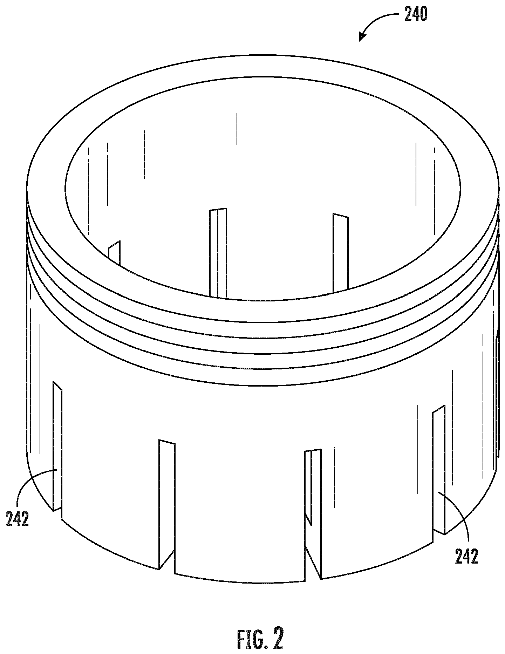

In some embodiments, the conductive portion of cover 140 may be configured to completely enclose the sensor electronics with a Faraday cage to protect the electronics from outside electromagnetic interference (EMI). The Faraday cage may also block signals inside the detector from radiating to the outside environment. The cover 140 includes one or more openings 142 for providing the Faraday cage. FIG.2 shows an example of cover 240, according to one or more embodiments. Openings 242 are in the form of slots. However, other shapes, sizes, and distribution may be considered and are compatible with the present disclosure. For example, one or more parameters (size, shape, and/or distribution) of the openings 242 may be chosen based on the electromagnetic signals (e.g., wavelengths) to be blocked while allowing light from the light indicator to pass through.

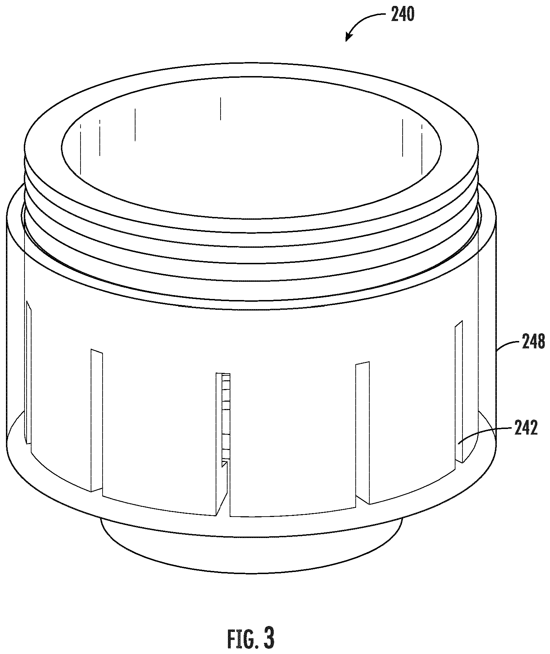

Returning to FIG. 1, in some embodiments, a portion of cover 140 may be configured to be covered with a layer of transparent (or relatively transparent) material 148 to protect sensor 150 while allowing status indicator lights to be visible from outside gas detector 100. This may help maintain ingress protection from water and dust while allowing gas to enter the sensor interface in the appropriate area. In some embodiments, the transparent layer may be overmolded over a portion of cover 140 (i.e., injection molding is used to add a layer of the transparent material to cover 140). In some embodiments, the layer of transparent material may be configured to cover the portion of cover 140 that includes openings 142. In some embodiments, layer 148 may be configured to cover the portion of cover 140 below threads 146. In addition to providing a better visual of the light indicators from outside the gas detector, overmolding the transparent layer may also provide time and cost savings. FIG. 3 shows an example of a cover 240 with a transparent layer 248.

In some embodiments, the transparent material used to add a layer to cover 140 may be chosen based on the one or more gas detector parameters (e.g., UV stability, chemical compatibility, thermal expansion and contraction stability, etc.). For example, the transparent material may include one or more of polymethyl methacrylate (PMMA), polycarbonate, acrylonitrile butadiene styrene (ABS), high density polyethylene (HDPE), nylon, polyetherimide (PEI), polyethylene terephthalate (PET), polypropylene (PP), and/or polystyrene (PS).

It should be understood that the description and the drawings are not intended to limit the invention to the particular form disclosed, but to the contrary, the intention is to cover all modifications, equivalents, and alternatives falling within the scope of the present invention as defined by the appended claims. Further modifications and alternative embodiments of various aspects of the invention will be apparent to those skilled in the art in view of this description. Accordingly, this description and the drawings are to be construed as illustrative only and are for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed or omitted, and certain features of the invention may be utilized independently, all as would be apparent to one skilled in the art after having the benefit of this description of the invention. Changes may be made in the elements described herein without departing from the scope of the invention as described in the following claims. Headings used herein are for organizational purposes only and are not meant to be used to limit the scope of the description.

As used throughout this application, the word "may" is used in a permissive sense (i.e., meaning having the potential to), rather than the mandatory sense (i.e., meaning must). The words "include", "including", and "includes" and the like mean including, but not limited to. As used throughout this application, the singular forms "a," "an," and "the" include plural referents unless the content explicitly indicates otherwise. Thus, for example, reference to "an element" or "a element" includes a combination of two or more elements, notwithstanding use of other terms and phrases for one or more elements, such as "one or more." The term "or" is, unless indicated otherwise, non-exclusive, i.e., encompassing both "and" and "or." Terms describing conditional relationships, e.g., "in response to X, Y," "upon X, Y,", "if X, Y," "when X, Y," and the like, encompass causal relationships in which the antecedent is a necessary causal condition, the antecedent is a sufficient causal condition, or the antecedent is a contributory causal condition of the consequent, e.g., "state X occurs upon condition Y obtaining" is generic to "X occurs solely upon Y" and "X occurs upon Y and Z." Such conditional relationships are not limited to consequences that instantly follow the antecedent obtaining, as some consequences may be delayed, and in conditional statements, antecedents are connected to their consequents, e.g., the antecedent is relevant to the likelihood of the consequent occurring. Further, unless otherwise indicated, statements that one value or action is "based on" another condition or value encompass both instances in which the condition or value is the sole factor and instances in which the condition or value is one factor among a plurality of factors. Unless otherwise indicated, statements that "each" instance of some collection have some property should not be read to exclude cases where some otherwise identical or similar members of a larger collection do not have the property, i.e., each does not necessarily mean each and every.

## Claims

1. An enclosure (101) for a gas detector (100), the enclosure configured to house a gas sensor (150), the enclosure comprising:
a housing (130), the housing configured to house a gas sensor (150), wherein the housing comprises first threads (114) on an inner wall (104) of the housing;
a cover (140; 240), the cover configured to be detachably connected to the housing, wherein the cover comprises
second threads (146) on an outer surface (144) for mating with the first threads;
**characterized in that** the cover (140; 240) further comprises:
a conductive portion comprising one or more openings (142; 242) and configured for providing a Faraday cage; and
a top layer (148; 248) covering the one or more openings and configured for guiding an indicator light from inside the enclosure to outside the enclosure.

2. The enclosure of claim 1, wherein the top layer (148; 248) is overmolded over a portion of the cover (140; 240).

3. The enclosure of claim 1 or 2, wherein the top layer comprises a transparent material (148; 248).

4. The enclosure of any preceding claim, wherein the top layer (148; 248) comprises one or more of polymethyl methacrylate (PMMA), polycarbonate, acrylonitrile butadiene styrene (ABS), high density polyethylene (HDPE), nylon, polyetherimide (PEI), polyethylene terephthalate (PET), polypropylene (PP), and/or polystyrene (PS).

5. A gas detector (100) comprising:
a gas sensor (150); and
the enclosure (101) of any preceding claim, the enclosure configured to house the gas sensor.

6. The gas detector of claim 5, wherein the gas sensor (150) is an intrinsically safe gas sensor having intrinsically safe designed control electronics.

7. A method of manufacturing a gas detector, the method comprising:
providing a gas sensor (150);
providing a housing (130), the housing configured to house the gas sensor (150), wherein the housing comprises first threads (114) on an inner wall (104) of the housing; and
providing a cover (140; 240), the cover configured to be detachably connected to the housing, wherein the cover comprises:
second threads (146) on an outer surface (144) for mating with the first threads;
**characterized in that** the cover (140; 240) further comprises:
a conductive portion comprising one or more openings (142; 242) and configured for providing a Faraday cage; and
a top layer (148; 248) covering the one or more openings and configured for guiding an indicator light from inside the enclosure to outside the housing.

8. The method of claim 7, further comprising overmolding the top layer (148; 248) over a portion of the cover (140; 240).

9. The method of claim 7 or 8, wherein the top layer (148; 248) comprises a transparent material.

10. The method of claim 7, 8 or 9, further comprising:
determining one or more parameters of the openings (142; 242) based on an electromagnetic signal to be blocked by the Faraday cage, the one or more parameters comprising size, shape, and/or distribution of the one or more openings.

11. The method of any of claims 7 to 10, wherein the top layer (148; 248) comprises one or more of polymethyl methacrylate (PMMA), polycarbonate, acrylonitrile butadiene styrene (ABS), high density polyethylene (HDPE), nylon, polyetherimide (PEI), polyethylene terephthalate (PET), polypropylene (PP), and/or polystyrene (PS).

12. The method of any of claims 7 to 11, wherein the gas sensor (150) is an intrinsically safe gas sensor having intrinsically safe designed control electronics.

## Patentansprüche

1. Hülle (101) für einen Gasdetektor (100), wobei die Hülle konfiguriert ist, um einen Gassensor (150) aufzunehmen, die Hülle umfassend:
ein Gehäuse (130), wobei das Gehäuse konfiguriert ist, um einen Gassensor (150) aufzunehmen, wobei das Gehäuse erste Gewinde (114) an einer Innenwand (104) des Gehäuses umfasst;
eine Abdeckung (140; 240), wobei die Abdeckung so eingerichtet ist, dass sie lösbar mit dem Gehäuse verbunden ist, wobei die Abdeckung Folgendes umfasst
zweite Gewinde (146) auf einer äußeren Oberfläche (144) zum Zusammenpassen mit den ersten Gewinden;
**dadurch gekennzeichnet, dass** die Abdeckung (140; 240) weiter Folgendes umfasst:
einen leitfähigen Abschnitt, der eine oder mehrere Öffnungen (142; 242) umfasst und zum Bereitstellen eines Faradayschen Käfigs konfiguriert ist; und
eine obere Schicht (148; 248), die die eine oder mehrere Öffnungen abdeckt und so konfiguriert ist, dass sie ein Indikatorlicht von der Innenseite der Hülle zur Außenseite der Hülle leitet.

2. Hülle nach Anspruch 1, wobei die obere Schicht (148; 248) über einen Abschnitt der Abdeckung (140; 240) gespritzt wird.

3. Hülle nach Anspruch 1 oder 2, wobei die obere Schicht ein transparentes Material (148; 248) umfasst.

4. Hülle nach einem der vorstehenden Ansprüche, wobei die obere Schicht (148; 248) eines oder mehrere von Polymethylmethacrylat (PMMA), Polycarbonat, Acrylnitril-Butadien-Styrol (ABS), Polyethylen hoher Dichte (HDPE), Nylon, Polyetherimid (PEI), Polyethylenterephthalat (PET), Polypropylen (PP) und/oder Polystyrol (PS) umfasst.

5. Gasdetektor (100), umfassend:
einen Gassensor (150); und
die Hülle (101) nach einem der vorstehenden Ansprüche, die Hülle zur Aufnahme des Gassensors konfiguriert ist.

6. Gasdetektor nach Anspruch 5, wobei der Gassensor (150) ein eigensicherer Gassensor ist, der eine eigensicher gestaltete Steuerelektronik aufweist.

7. Verfahren zur Herstellung eines Gasdetektors, das Verfahren umfassend:
Bereitstellen eines Gassensors (150);
Bereitstellen eines Gehäuses (130), wobei das Gehäuse so konfiguriert ist, dass es den Gassensor (150) aufnimmt, wobei das Gehäuse erste Gewinde (114) an einer Innenwand (104) des Gehäuses umfasst; und
Bereitstellen einer Abdeckung (140; 240), wobei die Abdeckung so konfiguriert ist, dass sie abnehmbar mit dem Gehäuse verbunden werden kann; wobei die Abdeckung Folgendes umfasst
zweite Gewinde (146) auf einer äußeren Oberfläche (144) zum Zusammenpassen mit den ersten Gewinden;
**dadurch gekennzeichnet, dass** die Abdeckung (140; 240) weiter Folgendes umfasst:
einen leitfähigen Abschnitt, der eine oder mehrere Öffnungen (142; 242) umfasst und zum Bereitstellen eines Faradayschen Käfigs konfiguriert ist; und
eine obere Schicht (148; 248), die die eine oder mehrere Öffnungen abdeckt und so konfiguriert ist, dass sie ein Indikatorlicht von der Innenseite der Hülle zur Außenseite des Gehäuses leitet.

8. Verfahren nach Anspruch 7, weiter umfassend das Umspritzen der oberen Schicht (148; 248) über einen Abschnitt der Abdeckung (140; 240).

9. Verfahren nach Anspruch 7 oder 8, wobei die obere Schicht (148; 248) ein transparentes Material umfasst.

10. Verfahren nach Anspruch 7, 8 oder 9, weiter umfassend:
Bestimmen eines oder mehrerer Parameter der Öffnungen (142; 242) basierend auf einem elektromagnetischen Signal, das durch den Faradayschen Käfig blockiert werden soll, wobei der eine oder die mehreren Parameter die Größe, Form und/oder Verteilung der einen oder mehreren Öffnungen umfassen.

11. Verfahren nach einem der Ansprüche 7 bis 10, wobei die obere Schicht (148; 248) eines oder mehrere von Polymethylmethacrylat (PMMA), Polycarbonat, Acrylnitril-Butadien-Styrol (ABS), Polyethylen hoher Dichte (HDPE), Nylon, Polyetherimid (PEI), Polyethylenterephthalat (PET), Polypropylen (PP) und/oder Polystyrol (PS) umfasst.

12. Verfahren nach einem der Ansprüche 7 bis 11, wobei der Gassensor (150) ein eigensicherer Gassensor ist, der eine eigensicher gestaltete Steuerelektronik aufweist.

## Revendications

1. Enceinte (101) pour un détecteur (100) de gaz, l'enceinte étant configurée pour loger un capteur (150) de gaz, l'enceinte comprenant :
un logement (130), le logement étant configuré pour loger un capteur (150) de gaz, dans lequel le logement comprend des premiers filetages (114) sur une paroi (104) interne du logement ;
un couvercle (140 ; 240), le couvercle étant configuré pour être relié de manière amovible au logement, dans lequel le couvercle comprend
de seconds filetages (146) sur une surface (144) externe pour s'accoupler aux premiers filetages ; **caractérisé en ce que** le couvercle (140 ; 240) comprend en outre :
une partie conductrice comprenant une ou plusieurs ouvertures (142 ; 242) et configurée pour fournir une cage de Faraday ; et
une couche (148 ; 248) supérieure couvrant les une ou plusieurs ouvertures et configurée pour guider un voyant lumineux de l'intérieur de l'enceinte vers l'extérieur de l'enceinte.

2. Enceinte selon la revendication 1, dans laquelle la couche (148 ; 248) supérieure est surmoulée sur une partie du couvercle (140 ; 240).

3. Enceinte selon la revendication 1 ou la revendication 2, dans laquelle la couche supérieure comprend un matériau (148 ; 248) transparent.

4. Enceinte selon une quelconque revendication précédente, dans laquelle la couche (148 ; 248) supérieure comprend un ou plusieurs parmi le polyméthacrylate de méthyle (PMMA), le polycarbonate, l'acrylonitrile butadiène styrène (ABS), le polyéthylène haute densité (HDPE), le nylon, le polyétherimide (PEI), le polyéthylène téréphtalate (PET), le polypropylène (PP), et/ou le polystyrène (PS).

5. Détecteur (100) de gaz comprenant :
un capteur (150) de gaz ; et
l'enceinte (101) selon une quelconque revendication précédente, l'enceinte configurée pour loger le capteur de gaz.

6. Détecteur de gaz selon la revendication 5, dans lequel le capteur (150) de gaz est un capteur de gaz à sécurité intrinsèque présentant une électronique de commande à sécurité intrinsèque.

7. Procédé de fabrication d'un détecteur de gaz, le procédé comprenant :
la fourniture d'un capteur (150) de gaz ;
la fourniture d'un logement (130), le logement configuré pour loger le capteur (150) de gaz, dans lequel le logement comprend des premiers filetages (114) sur une paroi (104) interne du logement ; et
la fourniture d'un couvercle (140 ; 240), le couvercle étant configuré pour être relié de manière amovible au logement, dans lequel le couvercle comprend
de seconds filetages (146) sur une surface (144) externe pour s'accoupler aux premiers filetages ; **caractérisé en ce que** le couvercle (140 ; 240) comprend en outre :
une partie conductrice comprenant une ou plusieurs ouvertures (142 ; 242) et configurée pour fournir une cage de Faraday ; et
une couche (148 ; 248) supérieure couvrant les une ou plusieurs ouvertures et configurée pour guider un voyant lumineux de l'intérieur de l'enceinte vers l'extérieur du logement.

8. Procédé selon la revendication 7, comprenant en outre le surmoulage de la couche (148 ; 248) supérieure sur une partie du couvercle (140 ; 240).

9. Procédé selon la revendication 7 ou la revendication 8, dans lequel la couche (148 ; 248) supérieure comprend un matériau transparent.

10. Procédé selon la revendication 7, la revendication 8 ou la revendication 9, comprenant en outre :
la détermination d'un ou plusieurs paramètres des ouvertures (142 ; 242) sur la base d'un signal électromagnétique à bloquer par la cage de Faraday, les un ou plusieurs paramètres comprenant la taille, la forme et/ou la distribution des une ou plusieurs ouvertures.

11. Procédé selon l'une quelconque des revendications 7 à 10, dans laquelle la couche (148 ; 248) supérieure comprend un ou plusieurs parmi le polyméthacrylate de méthyle (PMMA), le polycarbonate, l'acrylonitrile butadiène styrène (ABS), le polyéthylène haute densité (HDPE), le nylon, le polyétherimide (PEI), le polyéthylène téréphtalate (PET), le polypropylène (PP), et/ou le polystyrène (PS).

12. Procédé selon l'une quelconque des revendications 7 à 11, dans lequel le capteur (150) de gaz est un capteur de gaz à sécurité intrinsèque présentant une électronique de commande à sécurité intrinsèque.
